# EUROPÄISCHE PATENTSCHRIFT

(11) **EP 0 350 808 B1**
(45) Veröffentlichungstag und Bekanntmachung des Hinweises auf die Patenterteilung: **08.11.1995**
(21) Anmeldenummer: 89112440.6
(22) Anmeldetag: 07.07.1989
(51) Int. Cl.: C12Q 1/34, G01N 33/58, C07H 15/203

(54) **Verfahren zum Nachweis von Substanzen mit Hydrolaseaktivität**
Method for detecting substances with hydrolase activity
Méthode pour la détection de substances avec activité hydrolase

(30) Priorität: 11.07.1988 US 217383
(43) Veröffentlichungstag der Anmeldung: 17.01.1990
(73) Patentinhaber: BOEHRINGER MANNHEIM GMBH, 68298 Mannheim (DE)
(72) Erfinder: Herrmann, Rupert, Dr., D-8120 Weilheim (DE); Guder, Hans-Joachim, Dr., D-8120 Weilheim (DE); Güthlein, Werner, Dr., D-6800 Mannheim 24 (DE); Kuhr, Manfred, Dr., D-6800 Mannheim 31 (DE); Berger, Johann, Dr., Indianapolis, IN 46250 (US); Buck, Harvey, Dr., Indianapolis, IN 46250 (US)

(56) Entgegenhaltungen:
- EP-A- 0 025 227
- HISTOCHEMIE vol. 35, no. 3, 1973, Seiten 199 - 218; R. GOSSRAU: "Über denhistochemischen und mikrochemischen Nachweis der beta-Galactosidase mit 1-Naphthyl-beta-galactopyranosid"
- HISTOCHEMIE vol. 37, no. 1, 1973, Seiten 89 - 91; R. GOSSRAU: "Splitting ofnaphthol AS-BI beta-galactopyranoside by acid beta-galactosidase"
- ZEITSCHRIFT FÜR MIKROSKOPISCH-ANATOMISCHE FORSCHUNG vol. 95, no. 5, 1981,Seiten 721 - 765; E. STRAUSS ET AL: "Die Hydrolasen des Dünndarmepithels beimMeerschweinchen während der pränatalen Entwicklung"
- HISTOCHEMISTRY vol. 81, 1984, Seiten 321 - 330; T. LUND-HANSEN ET AL: "Aquantitative cytochemical assay of beta-galactosidase in single cultured humanskin fibroblasts"

## Beschreibung

Gegenstand der Erfindung ist ein Verfahren zum Nachweis von Substanzen mit Hydrolaseaktivität, ein zu seiner Ausführung geeignetes Mittel sowie neue Hydrolasesubstrate und ein Verfahren zu deren Herstellung.

Hydrolasen sind Enzyme, die in jüngerer Zeit große Bedeutung erlangt haben. Einerseits spielen sie eine wichtige Rolle im Stoffwechsel von Pflanzen, Tieren und auch des Menschen. Weicht die Konzentration einer Hydrolase in einem dieser biologischen Systeme von der normalerweise darin vorhandenen Konzentration ab, so kann dies die Ursache einer Krankheit sein. Daher ist es eine Aufgabe der klinischen Diagnostik, bei Vorliegen einer Krankheit durch Bestimmung der Konzentration einer Hydrolase in Körperflüssigkeiten eventuelle Abweichungen vom Normalwert festzustellen. Dies geschieht zweckmäßig über die Bestimmung der Hydrolaseaktivität mittels einer Indikatorreaktion. Die zu untersuchende Probe wird dazu mit einem Substrat für die betreffende Hydrolase versetzt. Die aus dem Substrat gebildete Menge an Produkt ist ein Maß für die vorhandene Hydrolasekonzentration.

Andererseits werden Hydrolasen immer häufiger als Enzyme zur Markierung von immunologisch aktiven Verbindungen eingesetzt. Besonders breite Anwendung hat hierbei β-Galactosidase zum Markieren von beispielsweise Antikörpern bei immunologischen Tests gefunden (Annals of Clinical Biochemistry 16, 221 (1979)). Tests dieser Art dienen dazu, den Gehalt eines immunologisch aktiven Analyten in einer Probe zu bestimmen. Sie sind so aufgebaut, daß die Konzentration des Analyten über einen passenden Immunpartner ermittelt wird, der als Markierung kovalent gebundene β-Galactosidase trägt. Durch die Testführung wird erreicht, daß die Konzentration des Immunpartners direkt abhängig ist von der Konzentration des zu bestimmenden Analyten. Die Konzentration des markierten Immunpartners wird ebenfalls über eine Indikatorreaktion sichtbar gemacht. In der Indikatorreaktion wird der mit β-Galactosidase markierte Immunpartner mit einem Substrat für β-Galactosidase umgesetzt.

Die gebildete Menge an Produkt ist proportional zu der Konzentration des Immunpartners. Durch Vergleich mit den Werten einer Eichkurve, die mit Hilfe von Proben mit bekannter Analytkonzentration aufgenommen wird, kann eine unbekannte Analytkonzentration in einer Probe bestimmt werden.

Hydrolasen werden auch als Enzyme zur Markierung von Nukleinsäuren in Verfahren zum Nachweis von Nukleinsäuren eingesetzt. Ein solches Verfahren, welches β-Galactosidase als Enzymmarkierung verwendet, ist in der DE-A-29 15 082 beschrieben. Auch hier wird die Menge an Markierung in einer Indikatorreaktion bestimmt.

Hydrolasen werden auch als Forschungsreagenzien verwendet. Auch hier ist es wichtig, die Konzentration der Enzyme genau und schnell bestimmen zu können. Im allgemeinen können hierzu ähnliche Methoden Einsatz finden, wie sie in der klinischen Diagnostik angewandt werden.

Das Substrat ist dabei meistens eine in der Probenflüssigkeit lösliche chemische Verbindung. Bei de Umsetzung mit der Hydrolase wird ein Produkt gebildet, welches sich in einer seiner Eigenschaften, beispielsweise einer charakteristischen Lichtabsorption, einer Lichtemission (Fluorenszenz) usw., vom Substrat unterscheidet und dadurch nachgewiesen werden kann.

Bei den Testverfahren auf immunologisch aktive Substanzen unterscheidet man zwei technische Ausführungsformen. Bei einer häufig verwendeten Ausführungsform findet die Nachweisreaktion in einer Küvette statt, wie sie für photometrische Untersuchungen allgemein verwendet wird. Die Auswertung der Indikatorreaktion wird dann durch Messung der Absorption, Emission oder Radioaktivität in einem geeigneten Gerät vorgenommen.

Bei einer anderen Ausführungsform laufen die Reaktionen in einem oder mehreren Vliesen oder Filmen, welche Teil eines Testträgers sind, ab. Auf diesen Vliesen oder Filmen sind die benötigten Reagenzien aufgebracht. Die Menge an Produkt, das durch die Hydrolaseaktivität aus dem Hydrolasesubstrat während der Indikatorreaktion freigesetzt wird, kann dann in einem solchen Vlies oder Film direkt ermittelt werden. Ein Vorteil dieser Ausführungsform ist, daß meist mit nur einer einzigen Lösung, der flüssigen Probe, gearbeitet werden kann, was die Verfahrensführung erheblich erleichtert. Besonders einfach läßt sich die Menge an gebildetem Produkt durch Messung der Lichtabsorption bei einer bestimmten Wellenlänge verfolgen.

Hierfür geeignete Substrate sind die in der EP-A-01 46 866 beschriebenen Phenolsulfonphthaleinyl-β-D-galactoside und ihre Derivate, aus denen durch Reaktion mit β-D-Galactosidase Phenol-sulfonphthaleinderivate freigesetzt werden. Während das Substrat eine gelbe Färbung aufweist, ist das Produkt rot gefärbt. Es findet also während der Indikatorreaktion ein allmählicher Farbumschlag von gelb über orange nach rot statt. Der Farbumschlag ist mit dem bloßen Auge nur sehr ungenau zu beurteilen, daher muß man zur Messung ein geeignetes Photometer benutzen.

Auch bei den in der EP-A-0 156 347 beschriebenen Resorufin-β-D-glycosiden handelt es sich um Substrate, bei deren Umsetzung mit einer Glykosidase ein Farbumschlag von gelb nach rot stattfindet. Es hat sich herausgestellt, daß im Übergangsbereich zwischen gelb und rot besonders bei niedrigen β-Glycosidaseaktivitäten die visuelle Auswertung zu subjektiven Ergebnissen führt, weshalb auch hier zur Auswertung Geräte eingesetzt werden. Diese Geräte sind jedoch relativ kompliziert und daher teuer. Die Resorufinglykoside sind als Substrate für den Einsatz in Testträgern nicht gut geeignet, da das aus ihnen gebildeten Resorufin ausblutet.

Hydrolasesubstrate, beispielsweise Methylumbelliferongalaktoside, bei deren Hydrolyse nur eine Änderung von Fluoreszenzeigenschaften stattfindet, sind ebenfalls für eine visuelle Auswertung ungeeignt. Auch sie führen zu Substanzen, die auf Teststreifen leicht ausbluten. Beispielsweise blutet das aus Methylumbelliferongalaktosiden gebildete Methylumbelliferon leicht aus.

Weiterhin wurden Verfahren zum Nachweis von Substanzen mit Hydrolaseaktivität vorgeschlagen, bei denen nicht gefärbte Hydrolasesubstrate eingesetzt werden, die bei der Reaktion mit einer Hydrolase in farbige Produkte übergehen. Bei diesen Nachweisreaktionen muß demnach nicht ein Farbumschlag, sondern das Entstehen einer Farbe beurteilt werden. Dies kann nämlich in einfacher Weise durch Vergleich der Farbintensität mit einem Farbton einer Farbskala geschehen. Auch farbenblinde Personen können einen solchen Test auswerten.

Ein solches Substrat ist 5-Brom-4-chlor-3-indolyl-β-galactosid. Dieses Substrat ergibt nach Spaltung und Oxidation einen Farbstoff, der auf Testträgern ausblutet. Das Substrat selbst ist aber wenig wasserlöslich und wird von β-Galactosidase nur langsam gespalten. Die entstehende Färbung ist daher nur sehr wenig intensiv. Ein darauf aufbauender Test ist für eine schnelle visuelle Auswertung somit nicht geeignet.

In Biochem. Z. 333, 209 (1960) wurden Verbindungen als Substrate vorgeschlagen, bei deren Hydrolyse Phenole freigesetzt werden. Diese bluten jedoch auf Testträgern leicht aus. Im Fall von Nitrophenol entwickelt sich während der Indikatorreaktion eine mehr oder weniger intensive Gelbfärbung. Die visuelle Auswertung im Bereich kleiner Konzentrationen ist bei dieser Wellenlänge sehr ungünstig.

Zur besseren Sichtbarmachung werden in einer zusätzlichen Reaktion die freigesetzten Phenole oxidativ mit Aminoantipyrin oder Methylbenzothiazolinonhydrazon gekoppelt (Anal. Biochem. 40, 281 (1971)) oder mit Diazoniumsalzen zu Azofarbstoffen (beispielsweise Histochemie 37, 89 (1973)) umgesetzt. Manche dieser Farbstoffe sind zwar für den Einsatz in Testträgern geeignet, da sie nicht so leicht ausbluten; der Einsatz dieser Kopplungsreaktion ist jedoch aus anderen Gründen von Nachteil. Zur Testführung auf einem Vlies oder einem Film müssen nämlich alle für diese zusätzliche Reaktion erforderlichen Reagenzien ebenfalls auf ein Vlies aufgebracht oder in einen Film eingebracht werden. Die große Anzahl von Reagenzien, wie beispielsweise Kopplungspartnern, kompliziert die Testführung und bringt zahlreiche Nachteile und Schwierigkeiten mit sich; so muß im Einzelfall darauf geachtet werden, daß die Reagenzien nicht schon vorzeitig miteinander oder unspezifisch mit anderen Komponenten der Probenlösung reagieren usw. So können Aminoantipyrin oder Methylbenzothiazolinonhydrazon auch mit Bestandteilen beispielsweise in untersuchtem Urin reagieren und so die Messung verfälschen. Andere Kopplungspartner, wie Diazoniumsalze, beeinträchtigen die Lagerstabilität. Auch ist bei der Wahl der Reagenzien zu berücksichtigen, daß sie nicht schon selbst eine Eigenfärbung aufweisen etc.

Ausblutungserscheinungen, die bei einigen der gebildeten Farbstoffe auf Testträgern auftreten, haben zum Beispiel zur Folge, daß die Farbintensität nicht an allen Stellen des Vlieses oder Films gleich ist, was zu Nachteilen bei der Auswertung führt. So werden beispielsweise die Meßwerte verfälscht.

In EP-A-0 025 227 und Histochemie 35 (1973), Seite 199-218 werden zum spezifischen Nachweis von 1-Naphtholen ebenfalls Kupplungsreagenzien benötigt:

Histochemie 35 (1973), Seite 199-218 beschreibt ein Nachweisverfahren für Galactosidase mit 1-Naphthyl-β-Galactopyranosid und einem Diazoniumkupplungsreagenz zur Farbbildung.

EP-A-0 025 227 beschreibt ein Nachweisverfahren für Analyte mit Hilfe eines H₂O₂-erzeugenden Systems und Umsetzung des H₂O₂ mit Peroxidase in Gegenwart eines Chromogens (u.a. 1-Naphthol oder 2,4-Dichlomaphthol) und einem Hydrazonkupplungsreagenz. Um eine vorzeitige oxidative Zersetzung des Chromogens zu verhindern, wird das Chromogen als ein Hydrolasesubstrat (z.B. 1-Naphthylphosphat) eingesetzt und reagiert erst nach Hydrolyse durch eine Phosphatase unter oxidativer Kupplung mit dem Kupplungsreagenz zu einem Farbstoff.

Es bestand daher ein Bedarf an einem Verfahren, bei dem die geschilderten Nachteile vermieden werden und mit dessen Hilfe Substanzen mit Hydrolase-Aktivität auch auf Testträgern in einfacher, schneller und zuverlässiger Weise nachgewiesen werden können.

Aufgabe der vorliegenden Erfindung war es, diesen Bedarf zu befriedigen.

Gelöst wird diese Aufgabe durch ein Verfahren zum Nachweis von Substanzen mit Hydrolase-Aktivität in einer Probe durch Mischen der Probe mit einem Hydrolasesubstrat sowie einem Oxidationsmittel ohne Verwendung eines zusätzlichen Kupplungsreagenzes und Auswertung der entstehenden Farbintensität, dadurch gekennzeichnet, daß als Hydrolasesubstrat mindestens eine Verbindung der Formel I 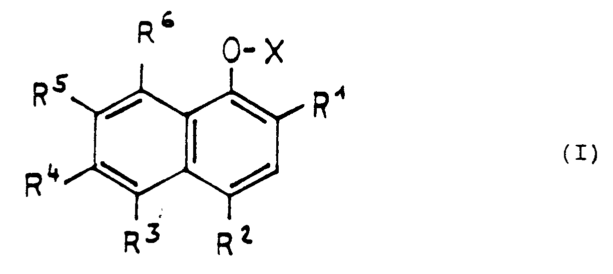
verwendet wird, in der
- R¹: Wasserstoff
- R²: eine C1-C4-Alkoxygruppe,
- R³, R⁴, R⁵ und R⁶,: die gleich oder verschieden sein können, Wasserstoff, eine Alkyl-, Alkoxy- oder Aralkoxygruppe, Halogen, eine Carboxyl-, Alkoxycarbonyl-, Alkylcarbonyl-, Carbamoyl-, Sulfo- oder Aminogruppe und
- X: ein Glykosyl-, Phosphat- oder Acylrest
bedeuten,
wobei die Reste R² und R³, R³ und R⁴, R⁴ und R⁵ sowie R⁵ und R⁶ jeweils zu einem Ringsystem geschlossen sein können,
Die Alkoxygruppen der Reste R², R³, R⁴, R⁵ und R⁶ bestehen bevorzugt aus 1 bis 4 Kohlenstoffatomen, die eine Kette bilden können oder verzweigt sind. Besonders bevorzugt sind der Methoxy-, der Isopropoxy-, der n-Propoxy- und der n-Butoxy-Rest.

Die Alkoxygruppen der Reste R², R³, R⁴, R⁵ und R⁶ können einoder mehrfach substituiert oder unsubstituiert sein. Als Substituenten sind bevorzugt Halogen und die Aminogruppe. Bevorzugte substituierte Reste sind der 1,1,1-Trifluor-eth-2-oxy- und der 1-Brom-eth-2-oxy-rest. Die Alkoxygruppen können auch die Gruppe O-(CH₂-CH₂-O)ₙ-R⁷ bedeuten, wobei n eine ganze Zahl zwischen 1 und 3 ist und R⁷ Wasserstoff, eine Alkylgruppe mit 1 - 4 C-Atomen, bevorzugt Methylgruppe, oder eine Verbindung der Formel I bedeutet.

Die Alkylgruppen der Reste R³, R⁴, R⁵ und R⁶ sind bevorzugt Kohlenwasserstoffreste mit 1 bis 4 Kohlenstoffatomen; besonders bevorzugt ist der Methylrest.

Die Aralkoxygruppe der Reste R³, R⁴, R⁵ und R⁶ umfaßt bevorzugt 1 - 4 Kohlenstoffatome im Alkylteil und 6 - 10 Kohlenstoffatome im Arylteil. Besonders bevorzugt ist der Benzyloxyrest.

Als Halogen in der Bedeutung der Reste R³, R⁴, R⁵ und R⁶ sind Fluor, Chlor, Brom und Jod zu verstehen, bevorzugt ist Chlor.

Alkylgruppen der Alkylcarbonylgruppe weisen bevorzugt 1 - 4 Kohlenstoffatome auf. Alkoxygruppen der Alkoxycarbonylgruppe weisen bevorzugt 1 - 4 Kohlenstoffatome auf.

Die Aminogruppen der Reste R³, R⁴, R⁵ und R⁶ sowie der Carbamoylgruppen können ein- oder zweifach substituiert oder unsubstituiert sein. Als Substituenten sind bevorzugt Acylreste und substituierte oder unsubstituierte Alkylreste. Besonders bevorzugt als Acylrest ist der Acetylrest.

Glykosylreste der Gruppe X sind Mono-, Oligo- und Polysaccharidreste der Hexosepyranosidreihe. Bevorzugt sind die Monosaccharidreste. Als Hexosepyranoside sind bevorzugt der Glukosid- und der Galactosidrest. Von diesen wiederum haben sich die β-Glykoside als besonders geeignet erwiesen.

Acylreste der Gruppe X weisen bevorzugt 1 bis 20 Kohlenstoffatome auf und können gesättigt oder ungesättigt, geradkettig oder verzweigt sein. Besonders bevorzugt ist der Acetylrest. Zu den Acylresten gehören auch Aminoacylreste. Bevorzugt als Aminoacylreste sind die über die Carboxylfunktion gebundenen Reste der natürlich vorkommenden α-L -Aminosäuren. Dabei können die freien polaren Reste der Aminosäuren, wie beispielsweise die Aminofunktionen, durch geeignete Schutzgruppen geschützt sein. Ein besonders bevorzugter Aminosäurerest ist der Alanylrest, der an der Aminofunktion einen Tosylrest trägt.

Der Phosphatrest ist der Rest -PO₃H₂ und seine Salze. Als Kationen in diesen Salzen können alle positiv geladenen Ionen dienen. Bevorzugt sind Alkali-, Erdalkali- und Ammoniumionen; besonders bevorzugt sind Natrium-, Kalium-, Magnesium- und Calciumionen.

In dem Falle, daß R² und R³ zu einem Ringsystem geschlossen sind, sind die Verbindungen bevorzugt, in denen Sauerstoffatome bzw. Aminogruppen der Reste R² und R³ so verbunden sind, daß sich ein 6gliedriger Ring ergibt. Bevorzugte Ringe, die so gebildet wurden, sind insbesondere der 2,2-Dimthyl-1,3-dioxin-ring und der 2,2-Dimethyl-3-oxazinring.

Im Fall, daß R³ und R⁴ oder R⁵ und R⁶ zu einem Ringsystem geschlossen sind, ist als Ringsystem ein aromatisches System bevorzugt. Besonders bevorzugt sind benzanellierte Verbindungen.

Für den Fall, daß R⁴ und R⁵ zu einem Ringsystem geschlossen sind, sind Ringsysteme mit 5 bis 6 Kohlenstoffatomen bevorzugt.

Die Verbindungen der Formal I können hergestellt werden durch Reaktion von Verbindungen der Formel II 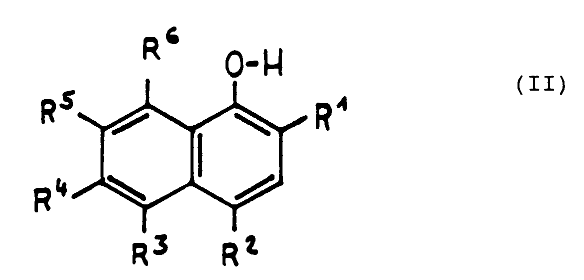
in denen R¹ bis R⁶ die oben angegebene Bedeutung haben, mit Verbindungen der Formel III

X-Y (III)

in denen X die oben angegebene Bedeutung hat und Y eine reaktive Gruppe bedeutet.

Für den Fall, daß X in Glycosylrest ist, muß dazu ein Verfahren angewandt werden, das eine Oxidation der stark oxidationsempfindlichen Aglykonkomponente II während der Umsetzung vermeidet. Klassische Verfahren, wie die Königs-Knorr-Reaktion oder davon abgeleitete Analoga, die als Reaktanden Silbersalze oder oxidativ wirkende Schwermetallsalze verwenden, können im vorliegenden Fall nicht eingesetzt werden, da die verwendeten Naphtholderivate sofort zum Farbstoff oxidiert würden.

Die Naphthole der Formel II werden mit einem aktivierten Zucker der Formel III in wässriger Alkalihydroxidlösung umgesetzt. Als besonders geeignet haben sich dabei Verbindungen erwiesen, bei denen Y eine gut austretende nucleophile Gruppe, beispielsweise Halogen, insbesondere Fluor, Chlor, oder Brom, oder ein Sulfonyloxyrest, insbesondere ein Toluolsulfonyloxy- oder Methansulfonlyoxyrest, bedeuten. Es hat sich dabei als zweckmäßig erwiesen, freie Hydroxygruppen des Zuckers oder Aminogruppen am Naphtholrest während der Reaktion durch eine geeignete Schutzgruppe geschützt zu halten.

Für den Fall, daß X ein Acylrest ist, können Verfahren angewandt werden, wie sie zur Herstellung von Estern aus Alkoholen sowie speziell Phenolen durch Reaktion mit organischen Carbonsäuren allgemein bekannt sind. Funktionelle Gruppen der Aminosäuren werden vor der Veresterung zweckmäßigerweise durch eine geeignete Schutzgruppe maskiert. Nach der Reaktion können die Schutzgruppen wieder abgespalten werden. Die reaktive Gruppe Y ist bevorzugt eine gut austretende nucleophile Gruppe. Vorteilhaft sind hierbei besonders Halogen, Alkoholat und Carboxylat. Beispielhaft beschrieben ist eine solche Hestellung in Liebigs Ann. Chem. 1984, 319-339 und in der EP-B-0 039 880.

Für die Herstellung von Verbindungen der Formel I, in denen X der Phosphatrest ist, sind besonders Verbindungen der Formel III geeignet, in denen X der Rest -POZ₂ oder -PZ₄, in denen Z eine gute Abgangsgruppe, insbesondere Alkoholat mit 1 - 4 C-Atomen oder Chlorid, Bromid oder Jodid bedeutet, und Y der Rest Halogenid, bevorzugt Chlorid, Bromid oder Jodid, oder Alkoholat bedeutet. Besonders bevorzugt als Verbindung der Formel X-Y ist Phosphoroxitrichlorid. Es ist zweckmäßig, freie Aminogruppen der Verbindung der Formel II durch nach der Reaktion abspaltbare Schutzgruppen zu maskieren.

Verbindungen, in denen X der Rest -POZ₂ oder -PZ₄ bedeutet, können durch Hydrolyse in bekannter Weise in die Phosphate überführt werden.

Die Salze der Phosphate können in bekannter Weise, beispielsweise durch Ionenaustausch, aus den Säuren hergestellt werden.

Die Verbindungen der Formel II und III sind bekannte Verbindungen oder können analog zu bekannten Verfahren der organischen Synthese hergestellt werden (siehe Liebigs Ann. Chem. 319 (1984), 2420 (1985), 1847 (1985), 251 (1985), 140 (1980), 297 (1987), 1321 (1980), Z. Naturforsch. 40b, 534 (1985), 41b, 377 (1986)). Insbesondere ist aus J. prakt. Chemie 62, 30 (1900) 4-Methoxy-1-naphthol bekannt, welches sich oxidativ zu einem blauen Farbstoff (das sogenannte Russig's Indigo) umsetzen läßt. Diese Oxidation wurde in Analytical Chemistry 36, 2494 (1964) zum Nachweis von Peroxidasen verwendet.

Obgleich also einige 4-Methoxy-1-naphthol-Derivate sowie die Möglichkeit, 4-Methoxy-1-naphthol oxidativ in einen Farbstoff umzuwandeln, seit vielen Jahren bekannt waren, wurden Methoxy-naphthol-Derivate bisher nicht als Hydrolasesubstrate in Verfahren zum Nachweis von Verbindungen mit Hydrolaseaktivität eingesetzt, in denen ohne Zusatz eines weiteren Kopplungspartners oxidativ die Bildung eines Farbstoffs bewirkt wird.

Überraschenderweise eignen sich die neuen Verbindungen der Formel I gemäß der vorliegenden Erfindung hervorragend als Hydrolasesubstrate. Sis sind wasserlöslich, farblos und stabil, solange sie nicht mit einer Hydrolase in Kontakt kommen. Ist eine außergewöhnlich gute Wasserlöslichkeit gewünscht, so verwendet man zweckmäßigerweise Verbindungen der Formel I, in denen einer oder mehrere der Reste R³, R⁴, R⁵ und R⁶ eine polare Gruppe, beispielsweise eine Carboxyl- oder Sulfogruppe bedeutet.

Verbindungen, bei denen durch Häufung lipophiler Reste die Wasserlöslichkeit des Substrats stark herabgesetzt ist, können ebenfalls als Hydrolasesubstrat eingesetzt werden, wenn man den Nachteil in Kauf nimmt, zur Erniedrigung der Polarität des Lösungsmittels in dem die Umsetzung stattfindet, beispielsweise geeignete organische Lösungsmittel oder Detergenzien, welche die Löslichkeit wieder erhöhen, zuzusetzen.

Als Hydrolasesubstrat wird eine Verbindung bezeichnet, wenn sie von der betreffenden Substanz mit Hydrolaseaktivität umgesetzt wird. Aus dem Substrat werden unter Aufnahme von Wasser zwei Produkte gebildet. Die Reaktion befolgt dabei die in der Enzymkinetik bekannten Regeln. Voraussetzung ist, daß die Substrate zumindest zu einem großen Anteil in der die Substanz mit Hydrolaseaktivität enthaltenden Lösung löslich sind.

Als Hydrolasesubstrate im erfindungsgemäßen Verfahren sind insbesondere diejenigen Verbindungen der Formel I geeignet, bei denen der Molekülteil X einem Molekülteil des natürlichen Substrats der entsprechenden Hydrolase analog ist. So sind beispielsweise als β-Galactosidasesubstrate die Verbindungen der Formel I, in denen X ein β-Galactosidrest bedeutet, als Esterasesubstrate die, in denen X einen Acylrest bedeutet und als Phosphatasesubstrat die Verbindungen der Formel I, in denen X ein Phosphatrest ist, besonders geeignet.

Substanzen mit Hydrolaseaktivität sind Substanzen, die chemische Verbindungen unter Verbrauch von Wasser in zwei Produkte spalten können. Dazu gehören natürlich vorkommende und künstlich erzeugte Hydrolasen der Enzymhauptklasse 3. Als besonders geeignet hat sich das erfindungsgemäße Verfahren zum Nachweis von Glykosidasen, bevorzugt Galactosidase, und Esterasen, bevorzugt Lipasen und Phosphatasen, erwiesen.

Unter Substanzen mit Hydrolaseaktivität werden auch Verbindungen dieser Hydrolasen mit anderen chemischen Verbindungen, beispielsweise immunologisch aktiven Verbindungen oder Nukleinsäuren verstanden. Zu den immunologisch aktiven Verbindungen zählen zum Beispiel Haptene, Antigene, Antikörper und Immunkomplexe, aber auch Fragmente von Antikörpern, wie das Fab- oder F(ab′)₂-Fragment. Die Verbindungen der Hydrolasen mit diesen immunologisch aktiven Verbindungen werden als Hydrolase-Konjugate bezeichnet. Die Hydrolase dient dabei zur Markierung der immunologisch aktiven Verbindung. Besonders geeignet ist das erfindungsgemäße Verfahren zum Nachweis von immunologisch aktiven Verbindung mit alkalischer oder saurer Phosphatase oder β-Galactosidase als Markierung.

Unter einer Probe wird hierbei bevorzugt eine feste oder flüssige Probe verstanden.

Im Falle einer festen Probe kann man zwischen löslichen und im wesentlichen nicht löslichen Proben unterscheiden.

Eine lösliche Probe wird zweckmäßig zum Nachweis in eine flüssige Probe überführt.

Unter einer im wesentlichen nicht löslichen Probe wird bevorzugt ein Feststoff verstanden, auf dessen äußerer oder innerer Oberfläche eine Substanz mit Hydrolaseaktivität gebunden ist. Die Art der Bindung ist für das erfindungsgemäße Verfahren nicht von Bedeutung. Diese Bindung kann beispielsweise sowohl kovalent als auch ionisch oder adsorptiv oder auch biospezifisch sein. Biospezifische Bindungen sind Bindungen zwischen biologischen Bindungspartnern, wie beispielsweise die Bindungen zwischen als Antigen oder Hapten wirkenden Substanzen und Antikörpern, Biotin und Avidin bzw. Streptavidin, komplementären Nukleinsäuren usw.

Unter einer flüssigen Probe, in der die Substanzen mit Hydrolase-Aktivität nachgewiesen werden sollen, werden im wesentlichen wässrige Lösungen verstanden. Diese Lösungen können beispielsweise Lösungen einer Hydrolase oder Hydrolasekonjugats in Wasser sein. Oft werden diesen Lösungen Beimengungen, wie Salze, Detergentien etc., beispielsweise zur Erhöhung der Lagerstabilität, zugegeben. Auch in solchen Lösungen kann das erfindungsgemäße Verfahren angewandt werden. Die flüssige Probe kann auch eine Körperflüssigkeit oder eine daraus durch Zugabe oder Abtrennung von Bestandteilen erhaltene Flüssigkeit sein. Dazu gehören beispielsweise Blut, Blutplasma, Serum oder Urin.

Die flüssige Probe kann bevorzugt auch eine Flüssigkeit sein, wie sie im Verlauf von immunologischen Testverfahren entsteht. Dieses sind beispielsweise die in Annals of Clinical Biochemistry 16, 221 (1979) beschriebenen und deren vorteilhafte Fortentwicklungen, die dem Fachmann auf dem Gebiet von Immunoassays bekannt sind. Auch in solchen Flüssigkeiten können Substanzen mit Hydrolase-Aktivität mit dem erfindungsgemäßen Verfahren nachgewiesen werden. Diese Lösungen enthalten meistens Puffersubstanzen, Stabilisatoren, Netzmittel etc., die den Nachweis jedoch nicht entscheidend stören.

Die Konzentration an Substanzen mit Hydrolase-Aktivität kann vorteilhaft in einem Bereich von 10⁻⁶ bis 10⁻¹⁵ mol/l, bevorzugt von 10⁻⁶ bis 10⁻¹² mol/l bestimmt werden.

Das Verfahren ist geeignet für Probelösungen, in denen der pH-Wert zwischen 5 und 11, vorzugsweise zwischen 6 und 10 liegt. Der optimale pH-Wert, bei dem das Verfahren durchgeführt wird, hängt von der verwendeten Hydrolase ab. Für die schnelle Durchführung des Verfahrens ist es zweckmäßig, bei oder in der Nähe des pH-Wertes zu arbeiten, bei dem die Hydrolase ein Aktivitätsmaximum aufweist. Außerhalb dieses Bereiches kann merkliche nicht enzymatische Hydrolyse bzw. Inaktivierung der Hydrolase stattfinden.

Die Nachweisreaktion wird bevorzugt in einem saug- oder quellfähigen Träger durchgeführt.

Solche Träger sind beispielsweise Vliese oder Filme. Bevorzugt sind Vliese. Unter Vliesen wird ein papierähnlich aus Fasern aufgebautes Material verstanden. Als Fasermaterial finden bevorzugt Cellulose, Kunststoffe oder Gemische davon Verwendung. Geeignet sind auch schwammartige oder/und poröse Träger.

Die Nachweisreaktion kann jedoch auch in einem Gefäß beliebiger Form, beispielsweise einer Küvette, durchgeführt werden. Wenn der Reaktionsverlauf mittels Absorptionsphotometer verfolgt werden soll, sind solche Verbindungen der Formel I besonders geeignet, bei denen Produkte gebildet werden, die im Reaktionsmedium löslich sind. Eine Möglichkeit ist die Verwendung von Verbindungen der Formel I, in denen je nach Polarität des Reaktionsmediums einer oder mehrere der Reste R³, R⁴, R⁵ und R⁶ eine polare Gruppe, beispielsweise eine Carboxyl- oder Sulfogruppe bedeuten. Eine weitere Möglichkeit zum Nachweis von Substanzen mit Hydrolaseaktivität in Lösung ist der Zusatz von die Polarität des Reaktionsmediums erniedrigenden Lösungsmitteln, wie beispielsweise organischen Lösungsmitteln oder Detergentien. Dadurch, daß man den Nachteil dieser zusätzlichen Komponente in Kauf nimmt, wird die Löslichkeit der Hydrolyseprodukte erhöht.

Für die Entwicklung einer Farbe während der Indikatorreaktion ist das Vorhandensein eines Oxidationsmittels erforderlich. Als Oxidationsmittel können solche Substanzen oder Substanzgemische dienen, deren Oxidationspotential oberhalb des Wertes für das freie Naphthol liegt. Als besonders vorteilhaft haben sich beispielsweise Kaliumhexacyanoferrat-III, Perborat/Peroxidase, Peroxid/Peroxidase, Tetrazoliumsalze oder Sauerstoff/Bilirubinoxidase erwiesen.

Zur Durchführung des erfindungsgemäßen Verfahrens wird die Probe mit dem entsprechenden Hydrolasesubstrat und einem Oxidationsmittel vermischt.

Die Vermischung der Probe mit dem Hydrolasesubstrat kann auf unterschiedliche Art und Weise erfolgen.

Das Vermischen der Probe mit dem Hydrolasesubstrat und dem Oxidationsmittel kann gleichzeitig oder nacheinander geschehen.

Im Falle einer flüssigen Probe, beispielsweise einer Lösung der Substanz mit Hydrolaseaktivität können das Hydrolasesubstrat bzw. das Oxidationsmittel beispielsweise in Form eines Feststoffs, beispielsweise eines Pulvers, einer Tablette, eines Lyophilisats u. ä., oder in Form einer Lösung der Probe zugesetzt werden.

Findet die Nachweisreaktion in einem saug- oder quellfähigen Träger statt, so hat es sich als besonders zweckmäßig erwiesen, die Probe auf einen Träger aufzugeben, auf den das Hydrolasesubstrat sowie gegebenenfalls das Oxidationsmittel und Zusatzstoffe imprägniert sind. Zur Imprägnierung wird eine Lösung der genannten Stoffe hergestellt und damit das Trägermaterial getränkt. Anschließend wird das getränkte Trägermaterial getrocknet.

Für den Fall, daß der Träger ein Film ist, werden bevorzugt das Hydrolasesubstrat sowie gegebenenfalls Oxidationsmittel und Zusatzstoffe schon bei dessen Herstellung in den Film inkorporiert.

Falls das Oxidationsmittel nicht zusammen mit dem Hydrolasesubstrat vorliegt, wird das Oxidationsmittel vor oder nach der Vermischung der Probe mit dem Hydrolasesubstrat mit der Probe vermischt.

Findet die Nachweisreaktion in einem Gefäß statt, so hat es sich als besonders zweckmäßig erwiesen, das Hydrolasesubstrat in fester Form oder als Lösung, evtl. vermischt mit dem Oxidationsmittel und mit Zusatzstoffen, einzusetzen. Auch hier können das Oxidationsmittel bzw. Zusatzstoffe getrennt zugegeben werden.

Im Fall einer festen Probe, beispielsweise wenn die Substanz mit Hydrolaseaktivität an ein Trägermaterial gebunden ist, ist es zweckmäßig, das Hydrolasesubstrat oder/und das Oxidationsmittel der Probe in Form einer Lösung zuzusetzen; es ist jedoch beispielsweise auch möglich, die Komponenten zunächst zu vermischen und dann eine Flüssigkeit zur Herstellung einer Lösung zuzugeben.

Wichtig für den Erfolg des Nachweisverfahrens ist, daß das Hydrolasesubstrat mit der in der Probe vorhandenen Substanz mit Hydrolaseaktivität so in Kontakt treten kann, daß die Enzymreaktion ablaufen kann.

Während der Indikatorreaktion bildet sich eine kräftige Farbe, die je nach Substitutionsmuster von rot über blau bis türkis reicht. Die Farbintensität kann nach bekannten Verfahren mit den Photometern, insbesondere auch Reflexionsphotometern, gemessen werden. Dazu wird Licht der Wellenlänge eingestrahlt, welches das Produkt der Indikatorreaktion absorbieren kann. Bevorzugt ist eine Wellenlänge zwischen 550 und 750 nm, besonders bevorzugt zwischen 600 und 700 nm.

Die Farbintensität wird mit den Werten einer Eichkurve verglichen, die man durch Messung der Werte für Proben mit bekannten Konzentrationen an Substanzen mit Hydrolase-Aktivität erhält, und jeder Intensität eine bestimmte Konzentration zuordnet. Dieser Vergleich wird zweckmäßig mit Hilfe eines Rechners vorgenommen.

Dadurch, daß kein Farbumschlag, sondern eine Farbentwicklung beobachtet werden muß, ist die Auswertung aber auch durch das menschliche Auge möglich. Dies hat den Vorteil, daß kein Gerät verwendet werden muß. So entfallen auch mögliche Fehlerquellen, wie falsche Bedienung des Geräts etc.

Das erfindungsgemäße Verfahren kann zum qualitativen, aber auch zum quantitativen Nachweis von Substanzen mit Hydrolaseaktivität angewandt werden. Zur qualitativen Auswertung wird beobachtet, ob sich eine Verfärbung ergeben hat. Zur quantitativen Bestimmung wird zu einem festgelegten Zeitpunkt visuell die Farbe mit einer Farbskala verglichen, die jeder Farbe eine bestimmte Konzentration zuordnet.

Das erfindungsgemäße Verfahren kann insbesondere auf Testträgern mit besonderen Vorteilen angewandt werden. Vorteile sind beispielsweise, daß das Verfahren einfach und billig ist und dennoch zuverlässige Ergebnisse liefert. Einfach ist das Verfahren beispielsweise dadurch, daß es auch visuell ausgewertet werden kann; dadurch entfallen teure Geräte. Es ist besonders zuverlässig, da es nicht zu Ausblutungserscheinungen auf Testträgern führt. Dies ist von großem Vorteil.

Unter Testträgern versteht man Mittel zum Nachweis von Substanzen durch Ausführung eines Tests. Sie bestehen üblicherweise hauptsächlich aus einer Grundplatte oder Folie, auf welcher die für den Test erforderlichen Reagenzien meist mittels Vliesen oder Filmen angebracht sind.

Im erfindungsgemäßen Verfahren werden keine weiteren Kopplungskomponenten, wie beispielsweise Diazoniumsalze, eingesetzt, da diese zu Nebenreaktionen bzw. Stabilitätsverlusten führen.

Bei immunologischen Testverfahren fallen ebenfalls Proben an, die auf Ihren Gehalt an Substanzen mit Hydrolaseaktivität, speziell von Hydrolasekonjugaten hin untersucht werden sollen.

Besondere Verwendung kann das erfindungsgemäße Verfahren zum Nachweis einer Substanz mit Hydrolaseaktivität daher beispielsweise in immunologischen Verfahren zum Nachweis eines Analyten finden. Solche Verfahren sind dem Fachmann auf dem Gebiet der Immunoassays bekannt (beispielsweise Annals of Clinical Chemistry 16, 221 (1979)). Diese Verfahren werden dahingehend abgewandelt, daß der darin erforderliche Nachweis der hydrolasemarkierten immunologisch aktiven Verbindungen mittels des erfindungsgemäßen Verfahrens durchgeführt wird. Beispielhaft seien erwähnt:

Für den Fall, daß der Analyt ein Antigen oder Hapten ist, haben sich folgende Verfahren bewährt:
- Die den Analyten enthaltende Probenlösung wird mit einem Überschuß an Konjugat aus einem Antikörper gegen den Analyten und aus einer Hydrolase versetzt. Es bildet sich ein Immunkomplex aus Analyt und Konjugat. Der Überschuß an Konjugat wird durch Immunreaktion an einen Träger gebunden, an den ein Überschuß an Analyt oder einer analytanalogen Verbindung gebunden ist. Die das Konjugat aus Hydrolase und Immunkomplex enthaltende Lösung wird vom Träger getrennt und gemäß dem erfindungsgemäßen Verfahren behandelt. Man erhält ein Versuchsergebnis für das Hydrolasekonjugat, aus welchem man auf die Anwesenheit bzw. auf die Menge des Analyten schließen kann. Eine andere Möglichkeit ist der Nachweis des an den Träger gebundenen Konjugats aus Hydrolase und Antikörper. Auch dieser ist nach dem erfindungsgemäßen Verfahren möglich.
- Die den Analyten enthaltende Probenlösung wird mit einem Überschuß von Konjugat aus einem Antikörper gegen den Analyten und einer Hydrolase versetzt. Es bildet sich ein Immunkomplex aus Analyt und Konjugat. Der Überschuß an Konjugat wird durch Immunreaktion an einen Träger gebunden, an den ein Überschuß eines weiteren Antikörpers gegen den Analyten gebunden ist. Die den Überschuß des Konjugats aus Hydrolase und Antikörper enthaltende Lösung wird vom Träger getrennt und nach dem erfindungsgemäßen Verfahren entweder das an den Träger gebundene Konjugat aus Immunkomplex und Hydrolase oder das in der Lösung enthaltene Konjugat aus Antikörper und Hydrolase nachgewiesen.
- Die den Analyten enthaltende Probenlösung wird mit einer bekannten Menge an Konjugat aus Analyt oder Analytanalogon und einer Hydrolase versetzt. Das Gemisch wird auf einen Träger aufgegeben, an welchen ein bekannter Unterschuß eines Antikörpers gegen den Analyten und das Analytanalogon bezogen auf die Summe aus Analyt und Konjugat gebunden ist.
   Ein Teil des Analyten und des Konjugats wird an den Träger gebunden. Nach dem erfindungsgemäßen Verfahren kann nach Abtrennung der Lösung entweder das an den Träger gebundene oder das in der Lösung befindliche Konjugat nachgewiesen werden.

Für den Fall, daß der Analyt ein Antikörper ist, können die gleichen Prinzipien angewandt werden, jedoch muß dann anstelle der Antikörper in den oben beschriebenen Verfahren ein Antigen oder ein gegen den Antikörper gerichteter Antikörper verwendet werden.

In immunologischen Verfahren zum Nachweis eines Analyten schließt sich an den Nachweis des Hydrolasekonjugats nach dem erfindungsgemäßen Verfahren die Auswertung an. Aus der Anwesenheit oder bei quantitativer Auswertung der Menge des nachgewiesenen Hydrolasekonjugats kann nämlich auf die Anwesenheit bzw. die Menge des Analyten in der ursprünglich eingesetzten Probe geschlossen werden. Dies kann beispielsweise über eine Eichkurve geschehen.

Weiter kann das erfindungsgemäße Verfahren zum Nachweis einer Substanz mit Hydrolaseaktivität in Verfahren zum Nachweis von Nukleinsäuren eingesetzt werden. Solche Verfahren sind dem Fachmann auf dem Gebiet der Nukleinsäurehybridisierungstests bekannt. In diesen Tests werden einsträngige oder doppelsträngige Nukleinsäuren, insbesondere DNA oder RNA oder Fragmente davon, die mit einer Hydrolase als Enzymmarkierung verbunden sind, nachgewiesen, deren Menge ein Maß für die Konzentration der nachzuweisenden Nukleinsäure ist. Der Nachweis dieser hydrolasemarkierten Nukleinsäuren erfolgt in vorteilhafter Weise gemäß dem erfindungsgemäßen Verfahren zum Nachweis einer Substanz mit Hydrolaseaktivität.

Die Vorteile dieses neuen immunologischen Verfahrens und des neuen Verfahrens zum Nachweis von Nukleinsäuren ergeben sich aus den Vorteilen des erfindungsgemäßen Verfahrens zum Nachweis von Substanzen mit Hydrolaseaktivität.

Ein weiterer Gegenstand der Erfindung ist ein Mittel zum Nachweis von Substanzen mit Hydrolaseaktivität, welches mindestens eine Verbindung der Formel I enthält. Ein solches Mittel ist zum Nachweis von Substanzen mit Hydrolaseaktivität in einer Probe geeignet, der in einem vorangehenden, gleichzeitigen oder nachfolgenden Schritt ein Oxidationsmittel zugesetzt wird.

Bevorzugt ist ein Mittel zum Nachweis von Substanzen mit Hydrolaseaktivität, welches mindestens eine Verbindung der Formel I und ein Oxidationsmittel enthält.

Weiterhin kann das Mittel Zusatzstoffe enthalten, die zwar für die eigentliche Nachweisreaktion nicht benötigt werden, jedoch eine vorteilhafte Wirkung ausüben. Dazu gehören insbesondere pH-Puffersubstanzen, die es erlauben, die Nachweisreaktion bei einem konstanten pH-Wert auszuführen. Ebenfalls werden davon umfaßt Netzmittel, welche eine gleichmäßige Benetzung von Trägermaterialien bewirken, und Detergentien.

Das erfindungsgemäße Mittel enthält bevorzugt einen oder mehrere saug- oder quellfähigen Träger. Solche Träger sind beispielsweise Vliese oder Filme, bevorzugt Vliese. Geeignet sind auch schwammartige oder poröse Träger.

Dieses Mittel enthält bevorzugt einen oder mehrere saug- oder quellfähige Träger, auf welche das Hydrolasesubstrat und das Oxidationsmittel gemeinsam bzw. getrennt imprägniert sind oder im Fall von Filmen inkorporiert sind. Die Herstellung solcher Mittel erfolgt nach an sich bekannten Verfahren.

Das erfindungsgemäße Mittel kann beispielsweise außer dem Hydrolasesubstrat und dem Oxidationsmittel sowie eventuell vorhandenen Zusatzstoffen auch ein Lösungsmittel enthalten. Bevorzugtes Lösungsmittel ist Wasser. Auch hier können Hydrolasesubstrat und Oxidationsmittel gemeinsam oder getrennt als Lösung vorliegen.

Das erfindungsgemäße Mittel kann auch in Form eines oder mehrerer Pulver oder Pulvermischungen vorliegen. Außer dem Hydrolasesubstrat und dem Oxidationsmittel kann ein solches Mittel bevorzugt übliche inerte, lösliche galenische Füllstoffe, wie Polyvinylpyrrolidon, Polyethylenglykole usw. enthalten. Die Pulver oder Pulvermischungen können auch zu Tabletten gepreßt sein.

Ein solches Mittel ist dazu geeignet, in einem Verfahren zum Nachweis von Substanzen mit Hydrolase-Aktivität eingesetzt zu werden.

Zum Nachweis von Substanzen mit β-Galactosidaseaktivität sind insbesondere Verbindungen der Formel IV geeignet, in denen X′ der β-Galactosidrest ist. Diese Verbindungen sind neu.

Ebenfalls Gegenstand der Erfindung sind daher Verbindungen der Formel IV 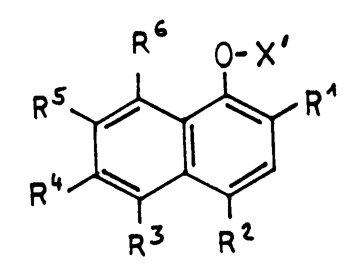
in der R¹, R², R³, R⁴, R⁵ und R⁶, die oben angegebene Bedeutung haben und X′ein β-Galactosidrest darstellt.

Diese Verbindungen zeichnen sich dadurch aus, daß sie in vorteilhafter Weise in dem erfindungsgemäßen Verfahren zum Nachweis von Substanzen mit β-Galactosidaseaktivität eingesetzt werden können, da es sich bei ihnen um β-Galactosidasesubstrate handelt.

Ein weiterer Gegenstand der Erfindung sind Verfahren zur Herstellung von Verbindungen der Formel IV, dadurch gekennzeichnet, daß eine Verbindung der Formel II 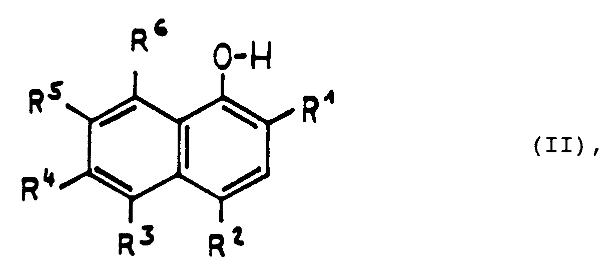
in denen R¹ bis R⁶ die oben angegebene Bedeutung haben,
mit einer Verbindung der Formel V

X′-Y (V),

in der X′ ein β-Galactosylrest ist und Y eine reaktive Gruppe bedeutet,
gegebenenfalls unter Verwendung von Schutzgruppen umgesetzt wird.

Ein weiterer Gegenstand der Erfindung ist die Verwendung von Verbindungen der Formel I 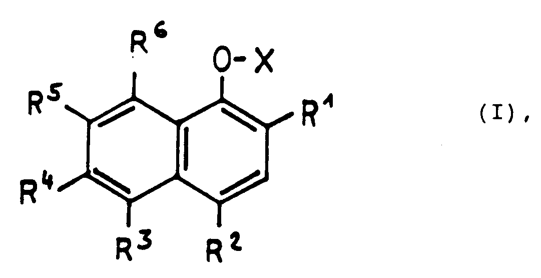
in welcher
- R¹: Wasserstoff
- R²: eine C1-C4-Alkoxygruppe,
- R³, R⁴, R⁵ und R⁶,: die gleich oder verschieden sein können, Wasserstoff, Halogen, eine Alkyl-, Alkoxy-, Aralkoxy-, Carboxyl-, Alkylcarbonyl-, Alkoxycarbonyl-, Carbamoyl-, Sulfo- oder Aminogruppe und
- X: ein Glykosyl-, Phosphat- oder Acylrest
bedeuten,
wobei die Reste R² und R³, R³ und R⁴ und R⁴ und R⁵ sowie R⁵ und R⁶ jeweils zu einem Ringsystem geschlossen sein können,
in den oben genannten Verfahren zum Nachweis von Substanzen mit Hydrolaseaktivität.

Die Erfindung wird durch die folgenden Beispiele weiter erläutert:

### Beispiel 1

### a) 4-Methoxy-1-naphthyl-2,3,4,6-tetra-O-acetyl-β-D-galactopyranosid

96.1 g (233.6 mmol) Acetobromgalactose (1-Brom-2,3,4,6-Tetra-O-acetyl-α-D-galactopyranose, Fluka) werden in 200 ml Aceton gelöst und durch Einleiten von N₂ deoxigeniert. Man erhitzt die Lösung unter permanentem Rühren zum Sieden und läßt zuerst eine Lösung von 14.3 g (254.9 mmol) Kaliumhydroxid in 13 ml Wasser und anschließend eine Lösung von 18.5 g (106.2 mmol) 4-Methoxy-1-naphthol (Liebigs Ann. Chem. 140 (1980)) in 200 ml Aceton jeweils innerhalb von 10 min zutropfen. Die Reaktionsmischung sollte während der Zugabe permanent unter Rückfluß kochen. Anschließend rührt man bei gleicher Temperatur noch 4 h, läßt abkühlen, filtriert von Ungelöstem ab und dampft das Filtrat im Hochvakuum ein. Der sirupöse Rückstand wird dreimal mit jeweils 100 ml Wasser digeriert und als Rohprodukt ohne weitere Aufreinigung in die nächste Synthesestufe eingesetzt.
DC (Kieselgel 60, Laufmittel Chloroform/Ethylacetat 20:1)
R_{F} = 0.40

### b) 4-Methoxy-1-naphthyl-β-D-galactopyranosid

Zu einer Suspension der Verbindung aus Beispiel 1 a) in 150 ml absolutem Methanol gibt man unter Feuchtigkeitsausschluß innerhalb von 1 h 25 ml einer 0.5 M Natriummethanolatlösung in absolutem Methanol so hinzu, daß der pH-Wert der Reaktionsmischung bei ca. 13 gehalten wird. Nach Abschluß der Reaktion (DC-Kontrolle) isoliert man das Produkt durch Säulenchromatographie an Kieselgel.
Ausbeute: 10 g
DC (Kieselgel 60, Laufmittel Chloroform/Methanol 3:1)
R_{F} = 0.40
¹H-NMR (DMSO-d₆): δ (ppm) = 3.41 - 3.80 (m, 6H); 3.92 (s, 3H); 4.52 - 4.89 (m, 4H); 5.31 (d, J = 5 Hz, 1H); 6.84 (d, J = 8.5 Hz, 1H); 7.16 (d, J = 8.5 Hz, 1H); 7.47 - 7.60 (m, 2H); 8.08 - 8.17 (m, 1H); 8.31 - 8.41 (m, 1H).
MS (pos. FAB): m/e = 337

### Beispiel 2

### 4-Propoxy-1-naphthyl-β-D-galactopyranosid

Aus 4-Propoxy-1-naphthol (Liebigs Ann. Chem. 140 (1980)) und Acetobromgalactose wurde analog zur Vorschrift aus Beispiel 1 a) und 1 b) 4-Propoxy-1-naphthyl-β-D-galactopyranosid hergestellt.
DC (Kieselgel 60, Laufmittel Chloroform/Methanol 3:1)
R_{F} = 0.42
¹³C-NMR (CD₃OD): δ (ppm) = 11.09, 23.78, 62.44, 70.30, 71.02, 72.55, 75.07, 76.94, 104.28, 105.39, 111.52, 122.65, 123.27, 128.45, 126.80, 127.61, 128.43, 148.51, 151.57
MS ((TMS)₄-Derivat): m/e = 652

### Beispiel 3

### 4-Isopropoxy-1-naphthyl-β-D-galactopyranosid

Aus 4-Isopropoxy-1-naphthol und Acetobromgalactose wurde analog zur Vorschrift aus Beispiel 1 a) und 1 b) 4-Isopropoxy-1-naphthyl-β-D-galactopyranosid hergestellt.
DC (Kieselgel 60, Laufmittel Chloroform/Methanol 3:1
R_{F} = 0.53
MS (neg. FAB): m/e = 363

### Beispiel 4

### 4-Trifluorethoxy-1-naphthyl-β-D-galactopyranosid

Aus 4-Trifluorethoxy-1-naphthol (Z. Naturforsch. 40b, 534 (1985)) und Acetobromgalactose wurde analog zur Vorschrift aus Beispiel 1 a) und 1 b) 4-Trifluorethoxy-1-naphthyl-β-D-galactopyranosid hergestellt.
DC (Kieselgel 60, Laufmittel Chloroform/Methanol 3:1)
R_{F} = 0.42
¹H-NMR (CD₃OD): δ (ppm) = 3.46 - 3.93 (m, 6H); 4.57 (q, J = 9 Hz, 2H); 4.86 (d, J = 6 Hz, 1H); 6.81 (d, J = 8 Hz, 1H); 7.09 (d, J = 8 Hz, 1H); 7.38 - 7.48 (m, 2H); 7.99 - 8.09 (m, 1H); 8.25 - 8.35 (m, 1H)

### Beispiel 5

### 4-Methoxy-1-naphthylphosphat-Dinatriumsalz

14.72 g (0.1 mol) 4-Methoxy-1-naphthol werden in 200 ml Pyridin gelöst und unter Rühren und Kühlung mit einem Eis-Kochsalzbad bei 3 °C 15.35 g (9.16 ml) (0.1 mol) Phosphoroxitrichlorid zugetropft. Nach 2 Stunden Rühren wird der gebildete kristalline Rückstand abgesaugt und das orangefarbene Filtrat in 400 ml Eiswasser eingetropft. Unter Zuhilfenahme einer Glaselektrode und eines pH-Meters stellt man durch Zutropfen von konz. Natronlauge auf pH 8 und engt im Vakuum am Rotationsverdampfer bei 50 °C zur Trockne ein. Man erhält 18 g braunes Harz; dieses wird in 100 ml Isopropanol heiß gelöst und durch Zugabe von 300 ml Diethylether wird das Produkt ausgefällt. Man erhalt 16.8 g 4-Methoxy-1-naphthylphosphat-Dinatriumsalz.
¹H-NMR (D₂O, NaOD):
δ (ppm) = 4.02 (s, 3H); 6.98 (d, J = 8.5 Hz, 1H); 7.38 (dd, J = 8.5 und 1.5 Hz, 1H), 7.61, 8.19 und 8.31 ppm (ABYZ-System, 2 und je 1H)

### Beispiel 6

### 1-Acetoxy-4-methoxynaphthalin

4-Methoxy-1-naphthol (1,75 g, 10 mMol) wird in Essigsäureanhydrid (18 ml) 2,5 Stunden lang am Rückfluß gekocht. Danach wird die Lösung im Vakuum eingeengt und der ölige Rückstand säulenchromatographisch (Kieselgel; Toluol/Essigester: 40/1) gereinigt. Nach Einengen der entsprechenden Fraktion wird das Rohprodukt aus Petrolether (Kp. 90-110°) umkristallisiert; Ausbeute 1,27 g (58 %); farblose Kristalle, Fp. 51-53°C.

### Beispiel 7

### Nachweis von menschlichem Choriongonadotropin (hCG) in einer Probe

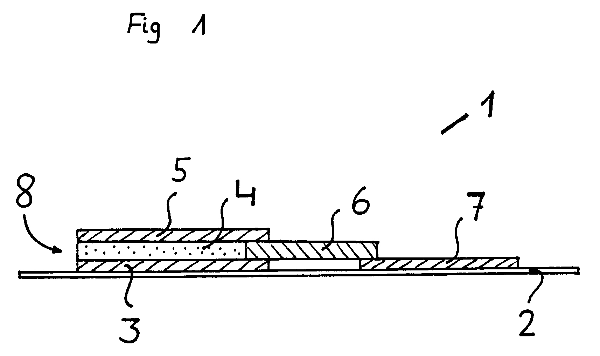

### A) Herstellung eines Testträgers 1 (Fig. 1)

### a) Herstellung von Vlies 3

Ein Stück Papier (Typ 4210 der Firma Kalff) wurde in einen Streifen (2,6 cm lang und 0,6 cm breit) geschnitten. 20 µl einer Lösung von 40 mg/ml 4-Methoxy-1-naphthyl-β-D-galactopyranosid (Galactosidasesubstrat) in DMSO wurden mit 20 µl einer 24 %igen Lösung von Polyvinylalkohol in Wasser vermischt. Diese Mischung wurde auf die Mitte des Papierstreifens aufgegeben.

### b) Herstellung von Vlies 5

Ein Stück Papier (Typ 4210 der Firma Kalff) wurde in einen Streifen (2,6 cm lang und 0,6 cm breit) geschnitten. Ein Ende wurde mit 100 µl PBS-Puffer (Phosphate buffered saline, pH 7,0, 1 % Rinderserum Albumin, 0,1 % Tween^{R}20) impragniert. Auf die Mitte des Streifens wurden 7,5 µl einer Lösung aufgegeben, die 20 U/ml eines Konjugats aus einem Fab-Fragment eines monoklonalen Antikörpers gegen hCG und β-Galactosidase (Substanz mit Galactosidaseaktivität), 100 µg/ml eines monoklonalen Maus-Antikörpers gegen die β-Kette von hCG und 7,5 mg/ml 4-Aminobenzyl-1-thio-β-D-galactopyranosid enthält. Das Ende, welches dem pufferimprägnierten Ende gegenüberliegt, wurde mit 10 µl einer 5 %igen Lösung von Polyvinylalkohol in Wasser imprägniert.

### c) Herstellung von Vlies 6

An ein Stück Papier (Typ 3512 der Firma Schleicher & Schüll) wurde nach Aktivierung mit Bromcyan Schafantikörper gegen den Fc-Teil von Mausantikörpern fixiert. Ein Stück dieses Materials wurde in einen Streifen (Länge 1,1 cm, Breite 0,6 cm) geschnitten.

### d) Herstellung von Vlies 7

Ein Stück Papier (Typ D-28 der Firma Whatman) wurde auf die Größe von 5 * 0,6 cm geschnitten.

### e) Vlies 3 und 5 sind durch eine Plastikfolie 4 voneinander getrennt.

Durch Aufkleben der getrockneten Vliese auf eine Grundfolie 2 mit der Breite 0.6 cm wurde der in Figur 1 dargestellte Testträger 1 hergestellt. Vlies 5 wird dabei so aufgeklebt, daß das mit Polyvinylalkohol imprägnierte Ende in Richtung des Endes 8 des Testträgers zeigt.

### B) Durchführung des Tests

### a) Probenvorbereitung

0,5 ml Probe wurden mit 0,5 ml einer Lösung, welche 4 mmol/l Natriumperborat und 10 mg/l Meerrettich-Peroxidase (Oxidationsmittel) in Phosphatpuffer pH 7 enthält, gemischt.

### b) Aufgabe der Probe auf den Teststreifen

Das Ende 8 des Teststreifens 1 wurde in die Lösung gestellt. Die hCG enthaltende Lösung drang in Vlies 5 und Vlies 3 ein und löste die darauf befindlichen Substanzen auf. Durch Reaktion von hCG mit dem β-galactosidasemarkierten Fab-Fragment eines monoklonalen Antikörpers gegen hCG und dem monoklonalen Antikörpers gegen die β-Kette von hCG bildete sich in der Lösung in Vlies 5 ein β-galactosidasemarkierter Immunkomplex aus den drei Komponenten. Dieser Immunkomplex, der in diesem Fall die Substanz mit Hydrolaseaktivität darstellt, wird nun mittels des erfindungsgemäßen Verfahrens in Vlies 6 nachgewiesen. Die Lösung mit dem Immunkomplex drang genauso wie die Lösung mit dem Substrat aus Vlies 3 in Vlies 6 ein und wurde dort mit dieser Substratlösung gemischt. In Vlies 6 wurde der gebildete Immunkomplex sowie der Überschuß des monoklonalen Antikörpers gegen die β-Kette von über die fixierten Schafantikörpers an Vlies 6 gebunden. Ein eventueller Überschuß an β-galactosidasemarkiertem Fab-Fragment floß weiter in Vlies 7. Durch die β-Galactosidasemarkierung des gebundenen Immunkomplexes und mit Hilfe des Oxidationsmittels bildete sich innerhalb von 10 Minuten eine blaue Farbe in Vlies 6.

Ist kein hCG in der Probe enthalten gewesen, so konnte sich auch kein β-galactosidasemarkierter Immunkomplex bilden. Daher wird keine Substanz mit Hydrolaseaktivität in Vlies 6 gebunden und somit keine Farbentwicklung gefunden. Hingegen ist die gesamte Menge an β-galactosidasemarkiertem Fab-Fragment noch in der Lösung enthalten, welche in Vlies 7 eindringt. Man findet daher in Vlies 7 eine Farbentwicklung, die von der Reaktion des β-galactosidasemarkierten Fab-Fragments herrührt, welches auch eine Substanz mit Hydrolaseaktivität ist. Somit findet eine Kontrolle des Tests dadurch statt, daß auch in Vlies 7 das erfindungsgemäße Verfahren durchgeführt wird; dies ist besonders in dem Fall wichtig, wenn kein hCG in der Probe vorhanden war. Die Auswertung kann auch quantitativ erfolgen, denn je mehr hCG in der Probe und somit gebundener β-galactosidasemarkierter Immunkomplex in Vlies 6 vorliegt, desto intensiver wird die Farbung in Vlies 6 nach Ablauf der 10 Minuten sein.

### Beispiel 8

### Nachweis von Thyroxin (T4)

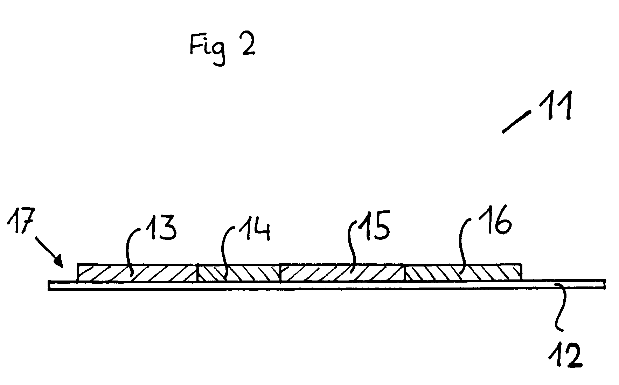

### A) Herstellung eines Teststreifens 11 (Fig. 2)

### a) Vlies 14

Vlies 14 ist mit einem Konjugat von β-Galactosidase mit einem Antikörper gegen T4 imprägniert.

### b) Vlies 15

Ein Stück Papier (Schleicher & Schüll 3512) wurde mit Bromcyan aktiviert und daran T4 Succinimidester gebunden.

### c) Vlies 16

Vlies 16 ist ein Vlies, welches mit 4-Methoxy-1-naphthyl-β-D-galactopyranosid imprägniert wurde.

### d) Vlies 13 dient zur Probenaufgabe.

Zur Herstellung des Testträgers 11 wurden die Vliese, wie in Figur 2 gezeigt, auf einer Grundfolie 12 befestigt.

### B) Durchführung des Tests

### a) Probenvorbereitung

Die Probe wurde analog zu Beispiel 7 vorbereitet.

### b) Durchführung

Der Teststreifen 11 wurde mit dem Ende 17 in die Lösung gestellt. Die T4-haltige Probe drang in Vlies 13 ein. Von dort floss sie weiter in Vlies 14, von wo sie das β-Galactosidase-Konjugat ablöste. Durch Immunreaktion des Antikörpers mit T4 bildete sich ein β-galactosidasemarkierter Immunkomplex. Die Lösung, welche nun neben diesen Immunkomplex noch einen Überschuß des β-Galactosidasekonjugats enthielt, drang in Vlies 15 ein. Dort wurde dieser Überschuß über gebundenes T4 an das Papier gebunden.

Die Lösung, welche noch den Immunkomplex enthält, drang in Vlies 16 ein. Dort findet das erfindungsgemäße Verfahren zum Nachweis des β-galactosidasemarkierten Immunkomplexes, welcher die Substanz mit Hydrolaseaktivitat darstellt, statt. Je mehr dieses Komplexes und somit auch von T4 in der Lösung enthalten war, desto stärker hatte sich Vlies 16 nach 10 Minuten blau gefärbt. Bei Abwesenheit von T4 blieb Vlies 16 weiß.

## Patentansprüche

1. Verfahren zum Nachweis von Substanzen mit Hydrolaseaktivität in einer Probe durch Mischen der Probe mit einem Hydrolasesubstrat sowie einem Oxidationsmittel ohne Verwendung eines zusätzlichen Kupplungsreagenzes und Auswertung der entstehenden Farbintensität, dadurch gekennzeichnet, daß als Hydrolasesubstrat mindestens eine Verbindung der Formel I, 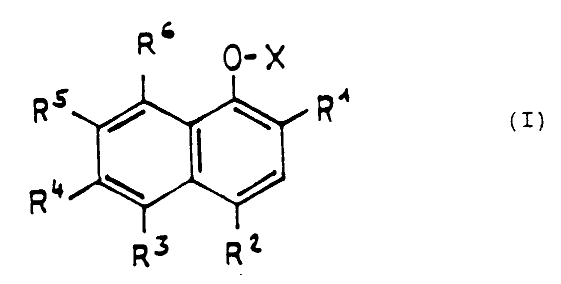 in welcher R1 Wasserstoff,
R2 eine C1- C4-Alkoxygruppe, R3, R4, R5 und R6, die gleich oder verschieden sein können, Wasserstoff, Halogen, eine Alkyl, Alkoxy-, Aralkoxy-, Carboxyl-, Alkylcarbonyl-, Alkoxycarbonyl-, Carbamoyl-, Sulfo- oder Aminogruppe und
X ein Glycosyl-, Phosphat- oder Acylrest bedeuten,
wobei die Reste R2 und R3, R3 und R4 und R4 und R5 sowie R5 und R6 jeweils zu einem Ringsystem geschlossen sein können,
verwendet wird.

2. Verfahren gemäß Anspruch 1, dadurch gekennzeichnet, daß R2 eine Methoxygruppe darstellt.

3. Verfahren gemäß Anspruch 1, dadurch gekennzeichnet, daß es in einem saug- oder quellfähigen Träger stattfindet.

4. Verfahren gemäß Anspruch 3, dadurch gekennzeichnet, daß die Auswertung visuell erfolgt.

5. Mittel zum Nachweis von Substanzen mit Hydrolaseaktivität in einer Probe, dadurch gekennzeichnet, daß es mindestens eine Verbindung der Formel I gemäß Anspruch 1 enthält.

6. Mittel gemäß Anspruch 5, dadurch gekennzeichnet, daß es einen oder mehrere saug- oder quellfähige Träger enthält.

7. Verbindungen der Formel IV, 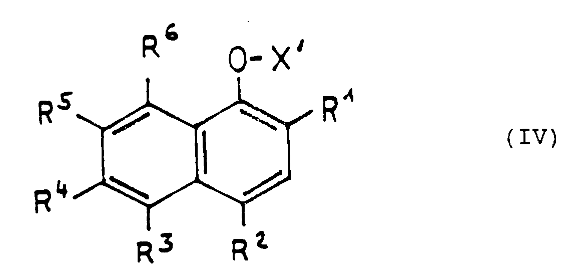 in welcher R1 Wasserstoff
R2 eine C1-C4-Alkoxygruppe
R3,R4, R5 und R6, die gleich oder verschieden sein können, Wasserstoff, Halogen, eine Alkyl-, Alkoxy-, Aralkoxy-, Carboxyl-, Alkylcarbonyl-, Alkoxycarbonyl-, Carbamoyl-, Sulfo- oder Aminogruppe und
X' ein β-Galaktosylrest bedeuten
und wobei die Reste R2 und R3, R3 und R4 und R4 und R5 sowie R5 und R6 jeweils zu einem Ringsystem geschlossen sein können.

8. Verbindungen gemäß Anspruch 6, dadurch gekennzeichnet, daß R2 eine Methoxygruppe darstellt.

9. Verfahren zur Herstellung von Verbindungen der Formel IV gemäß Anspruch 5, dadurch gekennzeichnet, daß eine Verbindung der Formel II 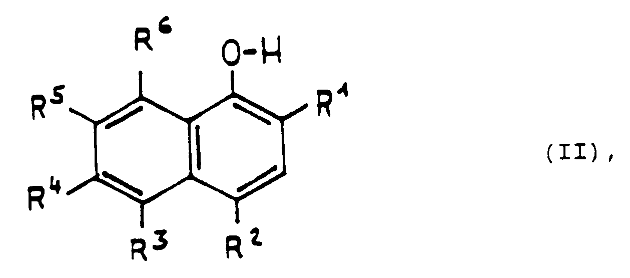 in der R1-R6 die in Anspruch 5 angegebene Bedeutung haben, mit einer Verbindung der Formel V
X'-Y (V),
in der X' ein β-Galaktosylrest ist und Y eine reaktive Gruppe bedeutet, gegebenenfalls unter Verwendung von Schutzgruppen umgesetzt wird.

## Claims

1. Process for the detection of substances with hydrolase activity in a sample by mixing of the sample with a hydrolase substrate, as well as an oxidising agent, without use of an additional coupling reagent, and evaluation of the resultant colour intensity, characterised in that, as hydrolase substrate, there is used at least one compound of the formula I 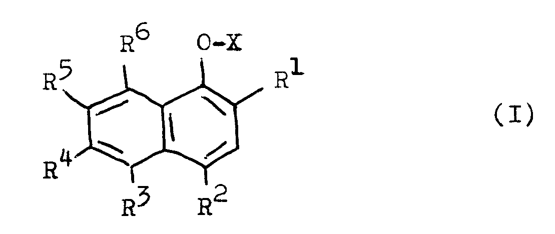 in which R¹ signifies hydrogen, R² a C₁-C₄-alkoxy group, R³, R⁴, R⁵ and R⁶, which can be the same or different, hydrogen, halogen, an alkyl, alkoxy, aralkoxy, carboxyl, alkylcarbonyl, alkoxycarbonyl, carbamoyl, sulpho or amino group and X a glycosyl, phosphate or acyl radical, whereby the radicals R² and R³, R³ and R⁴ and R⁴ and R⁵, as well as R⁵ and R⁶ can, in each case, be joined to give a ring system.

2. Process according to claim 1, characterised in that R² represents a methoxy group.

3. Process according to claim 1, characterised in that it takes place in an absorbent or swellable carrier.

4. Process according to claim 3, characterised in that the evaluation takes place visually.

5. Agent for the detection of substances with hydrolase activity in a sample, characterised in that it contains at least one compound of the formula I according to claim 1.

6. Agent according to claim 1, characterised in that it contains one or more absorbent or swellable carriers.

7. Compounds of the formula IV 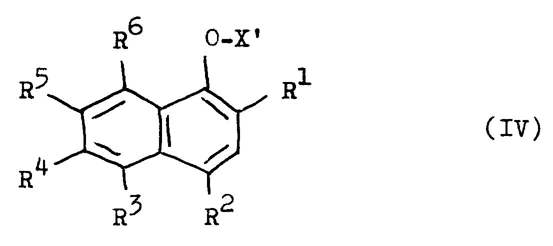 in which R¹ signifies hydrogen, R² a C₁-C₄-alkoxy group, R³, R⁴, R⁵ and R⁶, which can be the same or different, hydrogen, halogen, an alkyl, alkoxy, aralkoxy, carboxyl, alkylcarbonyl, alkoxycarbonyl, carbamoyl, sulpho or amino group, and X' a β-galactosyl radical and whereby the radicals R² and R³, R³ and R⁴ and R⁴ and R⁵, as well as R⁵ and R⁶ can in each case be joined to give a ring system.

8. Compounds according to claim 6, characterised in that R² represents a methoxy group.

9. Process for the preparation of compounds of the formula IV according to claim 5, characterised in that a compound of the formula II 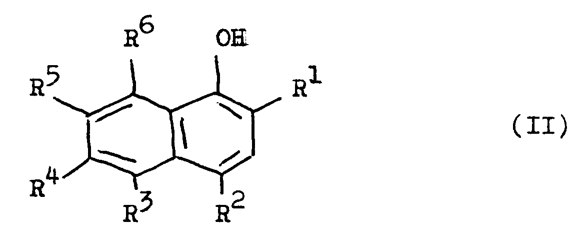 in which R¹ - R⁶ have the meaning given in claim 5, is reacted with a compound of the formula V
X' - Y (V)
in which X' signifies a β-galactosyl radical and Y a reactive group, possibly with the use of protective groups.

## Revendications

1. Procédé de détection, dans un échantillon, de substances ayant une activité d'hydrolase, par mélange de l'échantillon avec un substrat d'hydrolase ainsi qu'avec un agent oxydant, sans utilisation d'un réactif de couplage supplémentaire, et évaluation de l'intensité de coloration formée, caractérisé en ce qu'en tant que substrat d'hydrolase, on utilise un composé de formule I 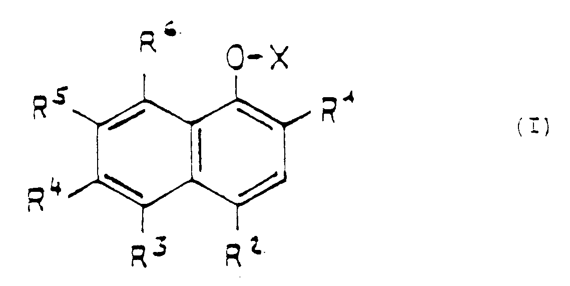 dans laquelle
R¹ représente un atome d'hydrogène,
R² représente un groupe alcoxy en C₁-C₄,
R³, R⁴, R⁵ et R⁶, qui peuvent être identiques ou différents, représentent chacun un atome d'hydrogène ou d'halogène, un reste alkyle, alcoxy, aralcoxy, carboxyle, alkylcarbonyle, alcoxycarbonyle, carbamoyle, sulfo ou amino, et
X représente un reste glycosyle, phosphate ou acyle,
les restes R² et R³, R³ et R⁴, R⁴ et R⁵ ainsi que R⁵ et R⁶ pouvant constituer chaque fois un système cyclique fermé.

2. Procédé selon la revendication 1, caractérisé en ce que R² représente un groupe méthoxy.

3. Procédé selon la revendication 1, caractérisé en ce qu'il est réalisé dans un support absorbant ou capable de gonfler.

4. Procédé selon la revendication 3, caractérisé en ce que l'évaluation est effectuée visuellement.

5. Moyen pour la détection de substances ayant une activité d'hydrolase, dans un échantillon, caractérisé en ce qu'il contient au moins un composé de formule I selon la revendication 1.

6. Moyen selon la revendication 5, caractérisé en ce qu'il contient un ou plusieurs supports absorbants ou capables de gonfler.

7. Composés de formule IV 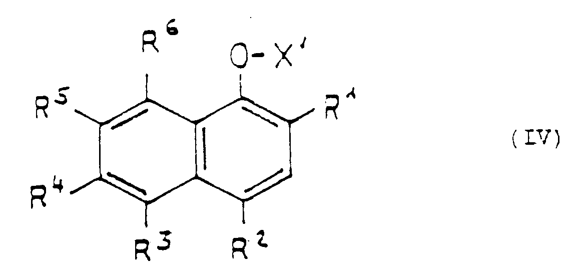 dans laquelle
R¹ représente un atome d'hydrogène,
R² représente un groupe alcoxy en C₁-C₄,
R³, R⁴, R⁵ et R⁶, qui peuvent être identiques ou différents, représentent chacun un atome d'hydrogène ou d'halogène, un reste alkyle, alcoxy, aralcoxy, carboxyle, alkylcarbonyle, alcoxycarbonyle, carbamoyle, sulfo ou amino, et
X' représente un reste β-galactosyle,
les restes R² et R³, R³ et R⁴, R⁴ et R⁵ ainsi que R⁵ et R⁶ pouvant constituer chaque fois un système cyclique fermé.

8. Composés selon la revendication 6, caractérisés en ce que R² représente un groupe méthoxy.

9. Procédé de préparation de composés de formule IV selon la revendication 5, caractérisé en ce que l'on fait réagir un composé de formule II 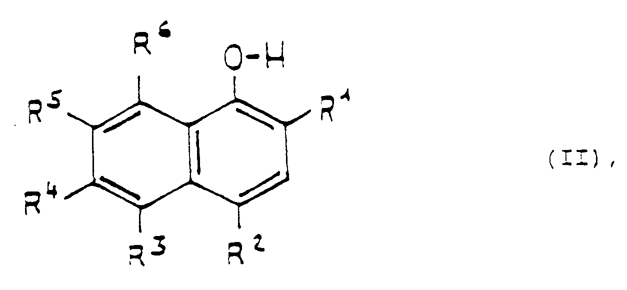 dans laquelle R¹-R⁶ ont les significations mentionnées dans la revendication 5, avec un composé de formule V
X'-Y (V),
dans laquelle X' représente un reste β-galactosyle et Y représente un groupe réactif, éventuellement en utilisant un groupe de protection.
